# FASCICULE DE BREVET EUROPEEN

(11) **EP 2 032 752 B1**
(45) Date de publication et mention de la délivrance du brevet: **26.10.2011**
(21) Numéro de dépôt: 07725367.2
(22) Date de dépôt: 18.05.2007
(51) Int. Cl.: D04H 1/40, A61F 13/15

(54) **DISPOSITIF DE FABRICATION DE NON-TISSES**
VORRICHTUNG ZUR HERSTELLUNG VON VLIESSTOFFEN
DEVICE FOR MANUFACTURING NONWOVENS

(30) Priorité: 18.05.2006 FR 0604537
(43) Date de publication de la demande: 11.03.2009
(73) Titulaire: Autoneum Technologies AG, 8406 Winterthur (CH)
(72) Inventeur: WEISKOPF, Charles, F-60000 Beauvais (FR)
(74) Mandataire: Van Adrichem Geisseler, Johanna
(86) Numéro de dépôt international: PCT/EP2007/004459
(87) Numéro de publication internationale: WO 2007/134812

(56) Documents cités:
- EP-A- 1 666 010
- WO-A-85/04366
- US-A- 4 005 957

## Description

La présente invention concerne un dispositif de fabrication d'une nappe de fibres non-tissées, un cylindre d'aspiration prévu pour être utilisé dans un tel dispositif, un procédé de fabrication d'une nappe de fibres non-tissées réalisée à l'aide d'un tel dispositif et une nappe de fibres non-tissées réalisée à l'aide d'un tel procédé.

L'industrie, et en particulier, l'industrie automobile, a un grand besoin de nappes réalisées à base de matériaux textiles non-tissés. Par exemple, l'insonorisation de l'intérieur d'un véhicule est réalisée à l'aide de telles nappes de non-tissés.

On connaît un dispositif de fabrication d'une nappe de fibres non-tissées capable de produire une nappe continue de fibres textiles et d'épaisseur constante.

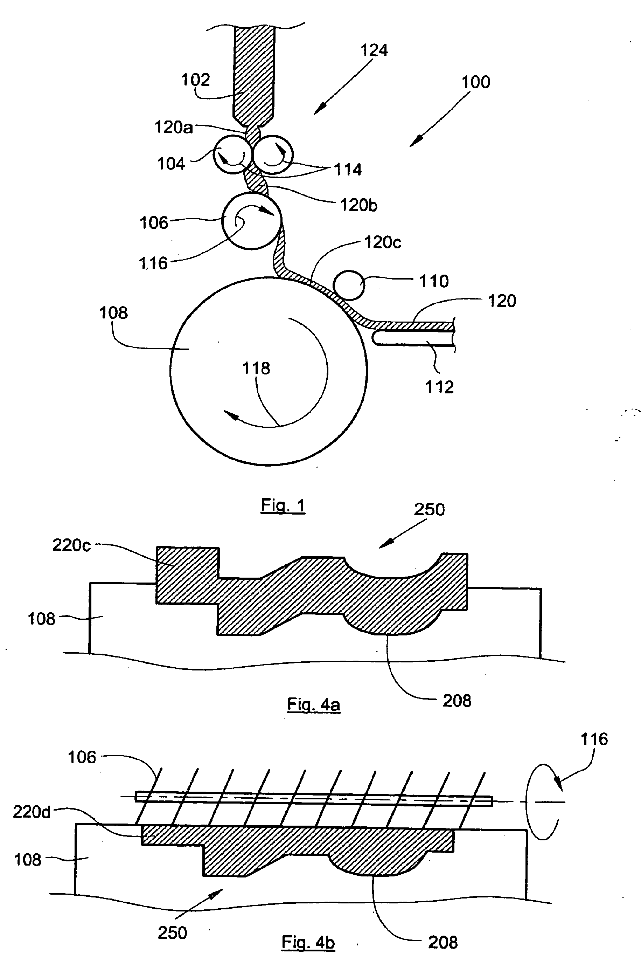

La Fig. 1 représente un tel dispositif de fabrication 100 qui comprend un dispositif d'alimentation en fibres 124, un cylindre d'aspiration 108, un rouleau de pression 110 et un tapis d'éjection 112.

Le dispositif d'alimentation en fibres 124 est prévu pour alimenter le cylindre d'aspiration 108 en fibres et il comprend un réservoir de fibres 102, un dispositif de répartition et une carde 106.

Les fibres sont constituées de fibres textiles ou synthétiques auxquelles a été ajouté un liant du type thermo-activable, comme par exemple une résine phénolique ou des fibres thermoplastiques. Ce liant permet, après un traitement approprié, de solidariser les fibres textiles entre elles afin que la nappe réalisée ait une certaine tenue mécanique.

Le principe de fonctionnement d'un tel dispositif de fabrication 100 est le suivant. En sortie du réservoir de fibres 102, les fibres 120a sont dirigées vers le dispositif de répartition qui est constitué de deux rouleaux 104 qui sont sensiblement tangents et qui tournent en sens inverse (flèche 114), ce qui permet de délivrer une quantité de fibres définie pour la suite du processus de fabrication. Les fibres 120b qui sortent du dispositif de répartition passent, alors, sur la carde 106 qui vient les ouvrir. La carde 106 est constituée d'un cylindre muni de pointes ou d'un fil denté enroulé qui tournent dans le sens de la flèche 116 et qui viennent aérer les fibres 120b. En sortie de la carde 106, les fibres 120b sont alors projetées sur le cylindre d'aspiration 108 qui tourne dans le sens de la flèche 118. La paroi extérieure du cylindre d'aspiration 108 est plane et percée d'une multitude de perforations. Un dispositif d'aspiration aspire l'air à l'intérieur du cylindre 108 à travers ces perforations. Les fibres 120b projetées sur la paroi du cylindre d'aspiration 108 sont, alors, plaquées contre la paroi du cylindre d'aspiration 108 réalisant, ainsi, un amalgame de fibres non-tissées 120c. Cet amalgame 120c passe ensuite sous le rouleau de pression 110 qui vient l'aplanir de manière à créer une nappe de fibres non-tissées 120 continue et d'épaisseur sensiblement constante. Cette nappe 120 est ensuite éjectée par le tapis d'éjection 112.

La nappe de fibres non-tissées 120 ainsi éjectée passe ensuite dans un dispositif de post-traitement qui la rigidifie. Un tel dispositif de post-traitement peut être, par exemple, un tunnel chauffant où de l'air chaud vient agir sur le liant.

La nappe de fibres non-tissées 120 ainsi rigidifiée est, ensuite, découpée en fonction des dimensions et de la forme définitive souhaitées et est moulée pour que la portion de nappe ainsi découpée prenne sa forme définitive.

Un tel dispositif de fabrication 100 génère des chutes importantes lors de la découpe. Ces chutes sont difficilement recyclables, ce qui augmente le prix de revient de chaque produit final.

En outre, pour que l'insonorisation d'un véhicule soit optimale, l'épaisseur de la nappe de fibres non-tissées doit être variable afin d'amortir au mieux les sons en fonction des positions des sources de bruits. Le procédé de fabrication de l'état de la technique ne permet pas de moduler facilement ni l'épaisseur, ni la forme du produit final obtenu.

Un dispositif de fabrication d'une nappe de fibres non-tissées et également connu du document WO - 85/04366.

Un objet de la présente invention est de proposer un dispositif de fabrication d'une nappe de fibres non-tissées comprenant :
- un dispositif d'alimentation en fibres ;
- une paroi d'aspiration conformée sous la forme d'un moule et alimentée en fibres par le dispositif d'alimentation en fibres, mobile relativement audit dispositif d'alimentation en fibres et munie de perforations ;
- un dispositif d'aspiration prévu pour aspirer l'air au travers desdites perforations de manière à plaquer les fibres contre la paroi d'aspiration et à réaliser une nappe ;
- un dispositif d'arasement prévu pour araser la face de la nappe orientée vers l'extérieur du moule ;
   le dispositif de fabrication étant **caractérisé en ce que** le dispositif d'alimentation en fibres comprend une carde et en ce que la carde constitue le dispositif d'arasement.

Avantageusement, le dispositif de fabrication d'une nappe de fibres non-tissées comprend un dispositif de récupération prévu pour récupérer les fibres arasées par le dispositif d'arasement.

Avantageusement, le dispositif de fabrication d'une nappe de fibres non-tissées comprend un dispositif de retraitement prévu pour renvoyer les fibres récupérées par le dispositif de récupération vers le dispositif d'alimentation en fibres.

Avantageusement, la paroi d'aspiration forme, au moins en partie, la paroi cylindrique d'un cylindre d'aspiration.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
la Fig. 1 représente un dispositif de fabrication d'une nappe de fibres non-tissées de l'état de la technique ;
la Fig. 2 représente un dispositif de fabrication d'une nappe de fibres non-tissées selon l'invention dans une position de moulage d'une nappe de fibres non-tissées ;
la Fig. 3 représente un dispositif de fabrication d'une nappe de fibres non-tissées selon l'invention dans une position de démoulage de la nappe de fibres non-tissées ;
la Fig. 4a représente une coupe du moule rempli ;
la Fig. 4b représente une coupe du moule au moment de l'arasement de la nappe de fibres ; et
la Fig. 5 représente un exemple d'une nappe de fibres non-tissées selon l'invention.

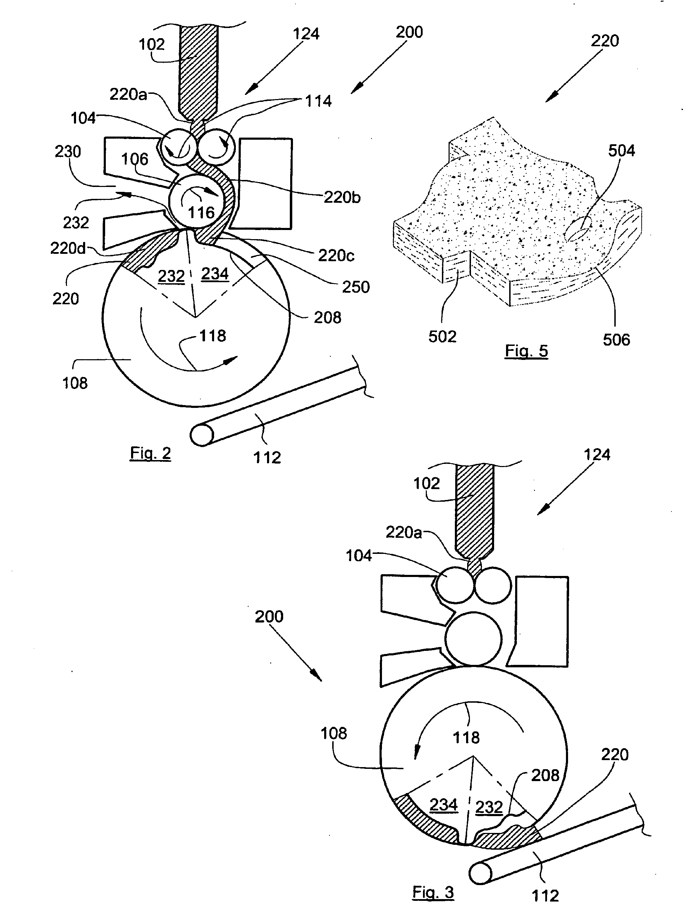

La Fig. 2 et la Fig. 3 représentent un dispositif de fabrication d'une nappe de fibres non-tissées 200 dont les éléments qui sont communs à ceux du dispositif de fabrication d'une nappe de fibres non-tissées 100 de l'état de la technique portent les mêmes références.

La Fig. 2 représente le dispositif de fabrication d'une nappe de fibres non-tissées 200 lors du moulage d'une nappe de fibres non-tissées 220 et la Fig. 3 représente le dispositif de fabrication d'une nappe de fibres non-tissées 200 lors du démoulage de la nappe de fibres non-tissées 220.

La Fig. 5 représente une nappe de fibres non-tissées 220 qui est délimitée par un contour 502 et qui comprend un évidement 504 ainsi qu'une réduction d'épaisseur 506.

Le dispositif de fabrication 200 comprend :
- un dispositif d'alimentation en fibres 12 4;
- une paroi d'aspiration 208 alimentée en fibres par le dispositif d'alimentation en fibres 124, qui est munie de perforations et est mobile relativement au dispositif d'alimentation en fibres 124 ;
- un dispositif d'aspiration prévu pour aspirer l'air au travers des perforations de manière à plaquer les fibres contre la paroi d'aspiration 208 et à réaliser une nappe 220.

Pour réduire les chutes de matériaux lors de la fabrication de la nappe de fibres non-tissées 220, la paroi d'aspiration 208 est conformée sous la forme d'un moule 250. La paroi d'aspiration 208 comprend un fond et des parois latérales qui s'étendent de manière sensiblement orthogonale au fond de manière à délimiter un volume que les fibres 220c viennent remplir de façon à former la nappe 220. La conformation sous forme du moule 250 permet de faciliter la réalisation de la nappe 220 et la forme du moule 250 est adaptée à la nappe 220 qui doit être obtenue. Le moule 250 présente ainsi des parois latérales de forme appropriée qui sont l'image de la nappe 220 devant être obtenue.

Afin d'obtenir une nappe 220 comprenant différentes épaisseurs, le fond de la paroi d'aspiration 208 n'est pas plan. En particulier, le moule 250 présente des creux et des bosses qui sont l'image de la nappe 220 devant être obtenue, c'est-à-dire que ces bosses et creux correspondent aux différentes épaisseurs à réaliser. Lorsqu'un trou doit être réalisé à un certain endroit dans la nappe 220, il suffit que le moule 250 possède un noyau à cet endroit, ce qui évite à des fibres de venir s'y loger. Contrairement au procédé de l'état de la technique, il n'est plus nécessaire de découper ce trou postérieurement à la réalisation de la nappe.

Ainsi dans le cas de la nappe 220 de la Fig. 5, les parois latérales du moule 250 prennent la forme du contour 502 et le fond du moule comprend un noyau qui permet de réaliser l'évidement 504 et une bosse qui vient réduire l'épaisseur de la nappe 220 au niveau de la réduction d'épaisseur 506.

Les perforations sont localisées sur le fond du moule 250 et éventuellement sur les parois latérales du moule 250.

Le dispositif d'alimentation en fibres 124 comprend un réservoir de fibres 102, un dispositif de répartition et une carde 106.

Le principe de fonctionnement du dispositif de fabrication 200 de l'invention est le suivant. En sortie du réservoir de fibres 102, les fibres 220a sont dirigées vers les deux rouleaux 104 tournant dans le sens des flèches 114 et constituant le dispositif de répartition. Les fibres 220b qui sortent du dispositif de répartition passent, alors, sur la carde 106 qui vient les ouvrir. En sortie de la carde 106, les fibres 220b sont alors projetées contre la paroi d'aspiration 208, c'est-à-dire à l'intérieur du moule 250 qui se remplit de fibres 220c.

Pour éviter que seul un endroit du moule 250 ne soit rempli, la paroi d'aspiration 208 se déplace par rapport au dispositif d'alimentation en fibres 124, permettant, ainsi, un remplissage total du moule 250. Ce déplacement est représenté par la flèche 118.

Dans un autre mode de réalisation, il est possible de prévoir que la paroi d'aspiration 208 soit fixe et que le dispositif d'alimentation en fibres 124 se déplace.

La vitesse de déplacement de la paroi d'aspiration 208 est asservie au débit de fibres afin d'assurer une densité préférée de fibres à l'intérieur du moule 250.

Dans le mode de réalisation de l'invention représenté sur les Figs., la paroi d'aspiration 208 s'étend sur un arc d'un cylindre d'aspiration 108. En d'autres termes, la paroi d'aspiration 208 forme, au moins en partie, la paroi cylindrique d'un cylindre 108. Du fait des dimensions du cylindre 108, il est possible de disposer une pluralité de parois d'aspiration 208 et donc de moules 250 sur sa paroi cylindrique. Un tel agencement permet de réaliser une pluralité de nappes de fibres non-tissées 220 pouvant être identiques ou différentes.

Le dispositif d'aspiration aspire l'air qui se situe à l'intérieur du moule 250 à travers les perforations de la paroi d'aspiration 208.

La Fig. 4a représente une coupe du moule 250 rempli de fibres 220c en amont d'un dispositif d'arasement et la Fig. 4b représente une coupe du moule 250 rempli de fibres 220c au moment de l'arasement.

Du fait du débit des fibres 220c dans le moule 250 au moment du remplissage, les fibres 220c viennent constituer la nappe 220 sous forme d'un matelas à épaisseur sensiblement constante (Fig. 4a).

Afin d'éliminer l'épaisseur de fibres 220c qui n'est pas nécessaire, un dispositif d'arasement est prévu pour araser la face de la nappe à épaisseur sensiblement constante 220 qui est orientée vers l'extérieur du moule 250. Le dispositif d'arasement est disposé en aval de l'endroit de remplissage du moule 250. Le dispositif d'arasement qui est, dans le mode de réalisation de l'invention décrit ici, constitué de la carde 106, comprend un ensemble de disques de coupe qui sont tangents à la face supérieure du moule 250. En l'espèce, la face supérieure du moule 250 correspond à la paroi extérieure du cylindre 108.

Le mouvement de rotation de la carde 106 vient donc projeter les fibres 220b dans le moule 250 et araser l'excédant de fibres 220c. Après arasement, les fibres 220c restantes constituent un amalgame 220d qui est maintenu dans le moule 250 par l'aspiration.

L'alimentation en fibres 220a se poursuit jusqu'au remplissage total du moule 250 et elle s'arrête lorsqu'il est rempli. La paroi d'aspiration 208 poursuit son mouvement de manière à positionner la nappe de fibres non-tissées 220 en vis-à-vis d'un dispositif de déchargement qui est, ici, constitué d'un tapis d'éjection 112. Dans la mesure où la nappe 220 n'est pas encore rigidifiée, il est préférable que l'éjection se fasse par la force de gravité, c'est pourquoi il est prévu que le mouvement de la paroi d'aspiration 208 s'accompagne d'un mouvement de retournement du moule 250. L'éjection de la nappe 220 se fait, alors, par arrêt de l'aspiration ou par une légère expiration à travers les perforations.

La nappe de fibres non-tissées 220 ainsi éjectée passe ensuite dans un dispositif de post-traitement qui la rigidifie. Comme dans le cas du dispositif de fabrication 100 de l'état de la technique, le dispositif de post-traitement peut être un tunnel chauffant où de l'air chaud vient agir sur le liant. La nappe de fibres non-tissées 220 ainsi rigidifiée est, ensuite, moulée sous pression à chaud pour qu'elle prenne sa forme définitive.

L'étape de découpe n'est alors plus nécessaire ce qui réduit les chutes et donc le prix de revient de chaque nappe 220.

Afin d'améliorer encore la rentabilité, le dispositif de fabrication 200 comprend un dispositif de récupération 230 prévu pour récupérer les fibres arasées par le dispositif d'arasement 106. La flèche 232 montre le chemin suivi par les fibres arasées.

Les fibres ainsi arasées n'ont subi aucun traitement particulier et peuvent donc être facilement ré-exploitées dans le dispositif de fabrication 200. A cet effet, il peut être prévu un dispositif de retraitement pour renvoyer les fibres récupérées par le dispositif de récupération 230 vers le dispositif d'alimentation en fibres 124.

Pour optimiser l'aspiration et/ou l'expiration au niveau de la paroi d'aspiration 208, le volume disposé sous la paroi d'aspiration 208 est divisé en une pluralité de secteurs 232 et 234. Ainsi, à l'aide d'un dispositif d'aspiration adapté, il est possible d'aspirer l'air au niveau de l'un des secteurs 234 tout en arrêtant l'aspiration ou en expirant de l'air au niveau de l'autre secteur 232. L'aspiration est, ainsi, réalisée de manière séquentielle à l'intérieur des secteurs 232 et 234.

Dans le cas du cylindre d'aspiration 108, l'intérieur du cylindre 108 est divisé en une pluralité de secteurs 232 et 234 et au moment de l'éjection de la nappe 220, il est possible de maintenir l'aspiration dans le secteur référencé 234 et de l'arrêter dans le secteur référencé 232, ce qui permet une dépose progressive de la nappe 220 sur le tapis d'éjection 112.

Comme cela est expliqué ci-dessus, le procédé de fabrication de la nappe de fibres non-tissées 220 comprend :
- une étape de présentation de la paroi d'aspiration 208 conformée sous la forme du moule 250 ;
- une étape de remplissage de la paroi d'aspiration 208 par des fibres 220c à l'aide du dispositif d'alimentation en fibres 124 par déplacement relatif de la paroi d'aspiration 208 par rapport au dispositif d'alimentation en fibres 124 ;
- une étape de moulage de la nappe 220 par plaquage des fibres 220c contre la paroi d'aspiration 208 par aspiration de l'air au travers de perforations prévues dans la paroi d'aspiration 208 ; et
- une étape de démoulage de la nappe 220 ainsi moulée.

Ce procédé permet d'obtenir des nappes 220 en trois dimensions du fait que les faces suivent les courbes du moule 250 et dont l'isodensité est garantie quelle que soit l'épaisseur de la nappe 220.

L'étape de démoulage de la nappe 220 est suivie d'une étape de moulage sous pression à chaud de la nappe 220, au cours de laquelle certaines parties de la nappe 220 sont plus ou moins écrasées afin de moduler la densité à certains endroits en fonction des sources de bruits.

Pour moduler l'épaisseur de la nappe 220, l'étape de moulage est suivie d'une étape d'arasement de la face de la nappe 220 orientée vers l'extérieur du moule 250.

Bien entendu, la présente invention n'est pas limitée aux exemples et modes de réalisation décrits et représentés, mais elle est susceptible de nombreuses variantes accessibles à l'homme de l'art.

## Revendications

1. Dispositif de fabrication d'une nappe de fibres non-tissées (200) comprenant :
- un dispositif d'alimentation en fibres (124) ;
- une paroi d'aspiration (208) conformée sous la forme d'un moule (250) et alimentée en fibres par le dispositif d'alimentation en fibres (124), mobile relativement audit dispositif d'alimentation en fibres (124) et munie de perforations ;
- un dispositif d'aspiration prévu pour aspirer l'air au travers desdites perforations de manière à plaquer les fibres contre la paroi d'aspiration (208) et à réaliser une nappe (220) ;
- un dispositif d'arasement (106) prévu pour araser la face de la nappe (220) orientée vers l'extérieur du moule (250) ;
le dispositif de fabrication (200) étant **caractérisé en ce que** le dispositif d'alimentation en fibres (124) comprend une carde (106) et **en ce que** la carde (106) constitue le dispositif d'arasement.

2. Dispositif de fabrication d'une nappe de fibres non-tissées (200) selon la revendication 1, **caractérisé en ce qu'**il comprend un dispositif de récupération (230) prévu pour récupérer les fibres arasées par le dispositif d'arasement (106).

3. Dispositif de fabrication d'une nappe de fibres non-tissées (200) selon la revendication 2, **caractérisé en ce qu'**il comprend un dispositif de retraitement prévu pour renvoyer les fibres récupérées par le dispositif de récupération (230) vers le dispositif d'alimentation en fibres (124).

4. Dispositif de fabrication d'une nappe de fibres non-tissées (200) selon une des revendications 1 à 3, **caractérisé en ce que** la paroi d'aspiration (208) forme, au moins en partie, la paroi cylindrique d'un cylindre d'aspiration (108).

## Claims

1. Device for manufacturing a fleece of non-woven fibres (200), comprising:
- a fibre supply device (124);
- a suction wall (208) which is shaped in the shape of a mould (250), is supplied with fibres by the fibre supply device (124), is movable relative to said fibre supply device (124) and is provided with perforations;
- a suction device provided to suck air through said perforations in such a way as to lay the fibres against the suction wall (208) and form a fleece (220);
- a levelling device (106) provided to level the facing of the fleece (220) pointing towards the outside of the mould (250);
the manufacturing device (200) being **characterised in that** the fibre supply device (124) comprises a card (106) and **in that** the card (106) forms the levelling device.

2. Device for manufacturing a fleece of non-woven fibres (200) according to claim 1, **characterised in that** it comprises a recovery device (230) provided for recovering the fibres levelled by the levelling device (106).

3. Device for manufacturing a fleece of non-woven fibres (200) according to claim 2, **characterised in that** it comprises a reprocessing device provided to return the fibres recovered by the recovery device (230) to the fibre supply device (124).

4. Device for manufacturing a fleece of non-woven fibres (200) according to any one of claims 1 to 3, **characterised in that** the suction wall (208) forms, at least in part, the cylindrical wall of a suction cylinder (108).

## Patentansprüche

1. Vorrichtung zur Herstellung einer Vliesstoffmatte (200), enthaltend:
- eine Faserzuliefereinrichtung (124);
- eine Ansaugwand (208), die in Form einer Pressform (250) ausgebildet ist und der über die Faserzuliefereinrichtung (124) Fasern zugeführt werden, wobei sie relativ zu der Faserzuliefereinrichtung (124) beweglich und mit Perforationen versehen ist;
- eine Ansaugeinrichtung, die dafür vorgesehen ist, die Luft durch die Perforationen so anzusaugen, dass die Fasern gegen die Ansaugwand (208) gepresst werden und eine Matte (220) bilden;
- eine Abstreicheinrichtung (106), die vorgesehen ist, um die zur Außenseite der Pressform (250) weisende Oberfläche der Matte (220) abzustreichen;
wobei die Vorrichtung zur Herstellung (200) **dadurch gekennzeichnet ist, dass** die Faserzuliefereinrichtung (124) eine Karde (106) aufweist, und dadurch, dass die Karde (106) die Abstreicheinrichtung bildet.

2. Vorrichtung zur Herstellung einer Vliesstoffmatte (200) nach Anspruch 1, **dadurch gekennzeichnet, dass** sie eine Rückgewinnungseinrichtung (230) aufweist, die dafür vorgesehen ist, die von der Abstreicheinrichtung (106) abgestreiften Fasern rückzugewinnen.

3. Vorrichtung zur Herstellung einer Vliesstoffmatte (200) nach Anspruch 2, **dadurch gekennzeichnet, dass** sie eine Wiederaufbereitungseinrichtung aufweist, die vorgesehen ist, um die von der Rückgewinnungseinrichtung (230) rückgewonnenen Fasern wieder zur Faserzuliefereinrichtung (124) zurückzuführen.

4. Vorrichtung zur Herstellung einer Vliesstoffmatte (200) nach einem der Ansprüche 1 bis 3, **dadurch gekennzeichnet, dass** die Ansaugwand (208) zumindest teilweise die zylindrische Wand eines Ansaugzylinders (108) bildet.
